(19) Europäisches Patentamt
European Patent Office
Office européen des brevets

(11) Publication number: 0 158 476
B1

(12) **EUROPEAN PATENT SPECIFICATION**

(45) Date of publication of patent specification: 23.01.91

(51) Int. Cl.[5]: C 07 D 489/08, C 07 D 489/02 // A61K31/485

(21) Application number: 85302103.8

(22) Date of filing: 26.03.85

(54) **Preparation of noroxymorphone from morphine.**

(30) Priority: 27.03.84 US 593744
20.03.85 US 713932

(43) Date of publication of application: 16.10.85 Bulletin 85/42

(45) Publication of the grant of the patent: 23.01.91 Bulletin 91/04

(84) Designated Contracting States: AT BE CH DE FR GB IT LI LU NL SE

(56) References cited:
DE-A-1 938 219
DE-C- 408 870
FR-A-2 395 269
FR-A-2 515 184

J.Heterocyclic Chem., vol. 14, 1977; p. 665

J.Med.Chem., vol. 24, 1981; pp. 1525-1528

**The file contains technical information submitted after the application was filed and not included in this specification**

(73) Proprietor: Mallinckrodt, Inc. (a Delaware corporation)
675 McDonnell Boulevard P.O. Box 5840
St. Louis Missouri 63134 (US)

(72) Inventor: Wallace, Rebecca A.
1444 Sunnytree Lane
Manchester, MO 63011 (US)

(74) Representative: Eyles, Christopher Thomas et al
BATCHELLOR, KIRK & CO. 2 Pear Tree Court
Farringdon Road
London, EC1R 0DS (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

**Description**

This invention relates to a method for preparing 14-hydroxy-3-0,N-(diethoxycarbonyl) normorphinone from 3-0,N-(diethoxycarbonyl) normorphinone dienol acetate and to a process for preparing noroxymorphone from morphine in high yield.

14-hydroxymorphinans, including such "nal" compounds as naloxone, naltrexone, and nalbuphine are important morphine derivatives due to their behavior as potent analgesics and/or narcotic antagonists. Prior to the present invention, among the most practical synthetic routes to the preparation of these pharmaceuticals have been processes which utilize thebaine as a starting material. In accordance with heretofore known processes, thebaine is oxidized to 14-hydroxycodeinone by use of m-chloroperbenzoic acid in an acetic acid/trifluoroacetic acid mixture or by a mixture of hydrogen peroxide and formic acid. 14-hydroxycodeinone is catalytically reduced to oxycodone which in turn is O-demethylated with boron tribrome to yield oxymorphone. After blocking of the hydroxyl groups with suitable blocking agents such as acetyl groups, the oxymorphone derivative is reacted with cyanogen bromide to yield an N-cyanodihydronormorphinone derivative which is thereafter hydrolyzed to 14-hydroxydihydronormorphinone (noroxymorphone), an important intermediate for preparation of naloxone, naltrexone and nalbuphine. However, such thebaine-bases syntheses are not entirely satisfactory for a number of reasons. For example, thebaine is in limited supply and its cost is high, thereby contributing to high cost of the noroxymorphone and the 14-hydroxymorphinans derived from it. Because of the scarcity and high cost of thebaine, efforts have been made in the art to devise methods for the synthesis of noroxymorphone and noroxycodone from compounds in more plentiful supply than thebaine.

Schwartz NL—A—8203204, published March 16, 1983, and FR—A—2,515,184, published April 29, 1983, provides such an alternative route to noroxycodone and noroxymorphone and the above "nal" compounds derivable therefrom. In the Schwartz method, oxidation of N-ethoxycarbonylnorcodeinone dienol acetate (derivable from codeine) with singlet oxygen affords 15-hydroxy-N-ethoxy-carbonyl-norcodeinone, which is thereafter converted to noroxymorphone via N-ethoxycarbonyl-noroxycodone. In the above Schwartz patent applications, there is disclosed a total synthesis or process for preparing noroxymorphone from codeine wherein (A) codeine is converted to N-ethoxycarbonyl-norcodeine, (B) the N-ethoxycarbonyl-norcodeine is converted to N-ethoxycarbonyl-norcodeinone, (C) the N-ethboxycarbonyl-norcodeinone is converted to N-ethoxycarbonyl-norcodeinone dienol acetate, (D) the dienol acetate is converted to 14-hydroxy-N-ethoxycarbonyl-norcodeinone, (E) the 14-hydroxy-N-ethoxycarbonyl-norcodeinone is converted to N-ethoxycarbonyl-noroxycodone, and (F) noroxymorphone is formed either (i) by converting the N-ethoxycarbonyl-noroxycodone to noroxycodone followed by converting the noroxycodone to noroxymorphone or (ii) by converting the N-ethoxycarbonyl-noroxycodone to N-ethoxycarbonyl-noroxymorphone followed by converting the N-ethoxycarbonyl-noroxymorphone to noroxymorphone. Further details of this method appear in an article by Schwartz et al, "Efficient Synthesis of 14-Hydroxymorphinans from Codeine", J. Med. Chem. (1981), *24*, pages 1525 to 1528.

Kavka, in copending U.S. Patent Application, Serial No. 593,752, assigned to the assignee hereof (now U.S. Patent No. 4472253), which corresponds to copending European patent Application No. 85302102.0 (EP—A—0159823), describes a method of preparing 14-hydroxy-N-ethoxycarbonyl-norcodeinone from N-ethoxycarbonyl-norcodeinone dienol acetate via oxidation with peroxy-acids and an improved proces for preparing noroxymorphone wherein the 14-hydroxy method is employed as Step D in total synthesis processes of the type disclosed in the above Schwartz applications.

However, there remains a substantial need in the art for a more efficient total synthesis process for preparing noroxymorphone from opium alkaloid morphinan compounds.

As indicated above, preparation of 14-hydroxycodeinone via oxidation of thebaine with m-chloroperbenzoic acid or other peroxy compounds has heretfore been known.

Hauser et al., "14-hydroxycodeinone. An Improved Synthesis", *Journal of Medicinal Chemistry* (1974), Vol. 17, no. 10, page 1117, states "14-hydroxycodeinone . . .

[Formula A]

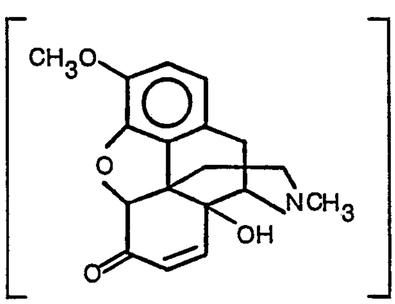

is normally prepared by oxidation of thebaine

[Formula B]

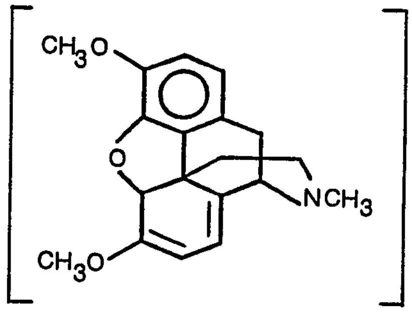

with either hydroben peroxide or potassium dichromate in acetic acid", citing M. Freund and E. Speyor, *J. Prakt. Chem., 94*(2), 135(1916). Hauser et al. also discloses synthesis of 14-hydroxymorphinan using an alternate procedure for the thebaine oxidation wherein m-chloroperbenzoic acid ("mClPBA") is added to a stirred solution of thebaine in a mixture of acetic acid and trifluoroacetic acid. The article reports 74% yield of product recrystallized from ethanol containing a small amount of chloroform. The total of the reported times for the various stages of the procedure is 75 minutes from initial m-chloroperbenzoic acid addition through a final stirring of the reaction mixture prior to cooling and pouring into ice water.

*Iijima, Rice and Brossi,* "The Oxidation of Thebaine with m-Chlorobenzoic Acid", *Helvetica Chimica Acta* (1977), Vol. 60, Fasc. 7-Nr. 213, pages 2135—37, states "The [above-mentioned]) procedure for oxidation of thebaine with . . . [mClPBA] reported by *Hauser et al.* is in our experience rather sensitive to small changes in the reaction conditions." Iijima et al. continues: "Using *shorter reaction times affords,* after extraction of the basified solution with chloroform and crystallization from ethanol, the unsaturated ketone *2* [*14-hydroxycodeinone*], however in *only 24% yield* instead of *the reported 74%*." The oxidation reaction conditions disclosed in the "Experimental part" of Iijima et al (pae 2136) include a total of 35 minutes from inmitial mClPBA addition through final stirring of the reaction mixture prior to cooling and pouring into ice water.

Introduction of β-hydroxy groups into 3-acetoxy- and 3-alkoxy-steroidal 3,5-dienes via reaction thereof with m-chlorperbenzoic acid has also heretofore been known. However, *aqueous* dioxan solvent has been deemed necessary to form the steroidal 6β-hydroxy-4-ene-3-ones in good yields from the acetoxy-steroidal 3,5-dienes.

*Kirk and Wiles,* "The Reaction of m-Chloroperbenzoic Acid with 3-Acetoxy-steroidal 3,5-Dienes," *Chemical Communications* (1970), page 518 ("Kirk$_I$") states that: "Enol Acetates (*cf.* I) [Formula C below

[Formula C]

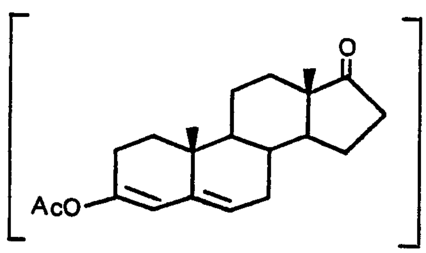

[Kirk$_I$ Formula I]

[3-acetoxyandrosta-3,5-diene-17-one]

or enol ethers of steroidal 4-en-3-ones are reported to give the corresponding 6 β-hydroxy-4-en-3-ones (II)

[Formula D]

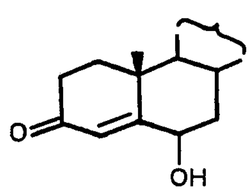

[Kirk$_I$ Formula II]

on reaction with peroxy-acids [citing J. Romo et al., *J. Org. Chem.* 1954, *19,* 1509; J. P. Dusza et al., *ibid.,* 1963, *28,* 92]. Yields, however, are generally low, and in our experience, not reproducible under the conditions described." Kirk$_{I}$ discloses that 3-acetoxyandrosta-3,5-diene-17-one of its formula I (Formula C above) reacts rapidly with mClPBA in solvents of low polarity (benzene, carbon tetrachloride, dichloromethane, *etc.*), with addition of OH and O·Co·$C_6H_4Cl$ groups on to the 5,6-double bond of the enol acetate, to form an unstable 1:1 addition compound of the formula

(Formula E)

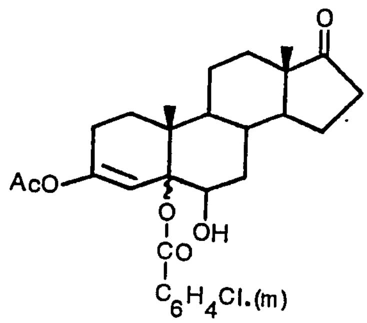

(Kirk$_{I}$ Formula III)

with little, if any, production of the 6β-hydroxy-steroid of its formula II (Formula D above).

Kirk$_{I}$ further discloses that "other steroidal 3-acetoxy-3,5-dienes (e.g., the enol acetate of cholest-4en-3-one) gave similar addition products with [mClPBA] . . . ;" in the absence of a 17-oxo group, 6β-hydroxy-4-en-3-ones were obtained in low and erratic yields; "However, the latter compounds were formed in good yields (up to 90%) if the reaction of any of these enol acetates with peroxy-acid was carried out in *aqueous* dioxan."

In *a subsequent Kirk and Wiles article,* "Competing Reactions in the Peroxyacid Oxidation of 3-Alkoxy-steroidal 3,5-Dienes," *Chemical Communications* (1970), pages 1015—1016 ("Kirk$_{I}$"), there is disclosed reaction between some dienol ethers of the formula

(Formula F)

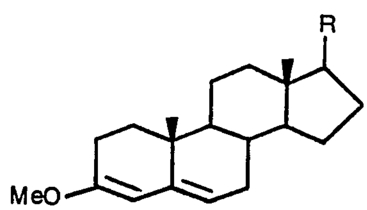

(Kirk$_{II}$ Formula II)

where: (a); R = $C_8H_{17}$
(b); R = B − OAc
(c); R = O

and peroxy-acid, using mClPBA. Kirk$_{II}$ discloses that in either the choletane series, Formula F (a), or androstane series, Formula F (b) or (c), the "major products were the 3,4-seco-aldehyde-ester" of the formula

(Formula G)

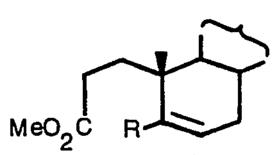

(Kirk$_{II}$ formula (IV))

where: (a); R = CHO
(b); R = $CH_2OH$
(c); R = $CO_2H$

"or the 6β-hydroxy-4-en-3-one" of the formula

(Formula H)

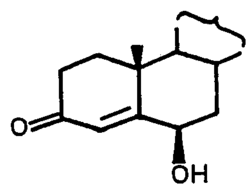

(Kirk$_{II}$ formula (III)

Kirk$_{II}$ adds: "Their proportions depended both upon the solvent and upon the method of mixing the reactants."; and "Anhydrous solutions (dioxan, carbon tetrachloride, dichloromethane, *etc.*) and immediate addition of an excess of peroxy-acid favour the aldehyde ester [Formula G (Kirk$_{II}$ Formula IVa) above] . . . , whereas *aqueous organic solvents,* and *gradual addition of peroxy-acid* to the steroid, favour the *6B-hydroxy compound* [Formula H (Kirk$_{II}$ formula III) above]."

Kirk$_{II}$ further discloses (at p. 1016) that the dienol ether (Formula F, Kirk$_{II}$ formula II, above) is oxidized preferentially at the 3,4-bond in the absence of water, but at C-6, when water is present; that such behavior is "almost unique among the known reactions of 3,5-dienol ethers (and esters), which are normally attacked at C-6, by electrophilic reagents. [citing R. Gardi et al., *J. Org. Chem.,* 1967, *32,* 2647 at 'ethers' and D. N. Kirk et al., 'Steroid Reaction Mechanisms,' Elsevier, Amsterdam, 1968, poage 184 at 'esters'.]"

Reaction of morphine and phenyl chloroformate with a view to producing normorphine is described by Kenner C. Rice et al, "Procedural Refinements in the N-Demethylation of Morphine and Codeine Using Phenyl Chloroformate and Hydrazine", J. Het. Chem., *14* (1977), pages 665 to 666.

FR—A—2395269 and GB—A—2000137 describe preparation of noroxymorphone from the compound of the following general formula:

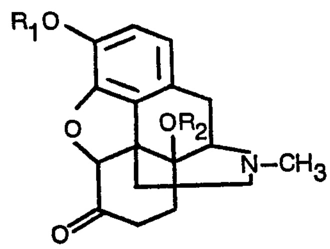

in which $R_1$ is a hydrogen atom or an alkyl radical containing up to 4 carbon atoms or an aralkyl radical containing 7 to 9 carbon atoms or an alkoxyalkyl radical containing 2 to 6 carbon atoms or a lower aliphatic or an aromatic acyl radical and $R_2$ is a hydrogen atom or a lower aliphatic or an aromatic acyl radical.

It has surprisingly now been found that 14-hydroxy-3-O,N-(diethoxycarbonyl) normorphinone can be repeatedly formed in good yields at fast rates and with good reliability by contacting 3-O,N-(diethoxycarbonyl)-normorphinone dienol acetate with peroxy-acid compounds in the substantial absence of water. It has also surprisingly now been found that such 14-hydroxy compound can be converted with such attributes to noroxymorphone, and that noroxymorphone can be prepared from morphine in even higher overall total-synthesis yield that is attainable by the codeine-to-noroxymorphone process described in the above Kavka application. The present invention substantilly fulfils the above-mentioend need for a more efficient process for preparing noroxymorphone from an opium alkaloid morphinan. It also substantially fulfils the need for a novel, efficient method of introducing a 14β-hydroxy group into 3-O,N-disubstituted normorphinone dienol acetate.

Generally stated, the present invention provides a process for preparing noroxymorphone from morphine which comprises:

(A) converting morphine having the formula (I)

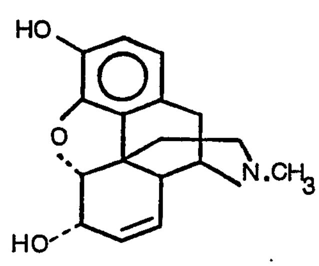

I

by reaction with a haloformate ester of the formula X—C(=O)OR$_1$, wherein R$_1$ is a C$_1$ to C$_7$ alkyl, 1,1,1-trichloroethyl, butenyl, benzyl, phenyl or naphthyl group and X is a halogen atom, to a 3-O,N-(di-R$_1$-oxycarbonyl) normorphine having the formula (J)

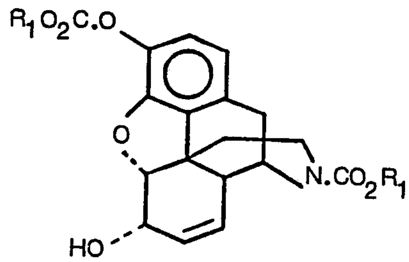

J

wherein R$_1$ is as defined above;

(B) oxidizing the 3-O,N-(di-R$_1$-oxycarbonyl) normorphine having the formula (J) by reaction with an oxidizing agent effective for oxidizing allylic —OH groups to keto groups to form a 3-O,N-(di-R$_1$-oxycarbonyl)-normorphinone having the formula (K)

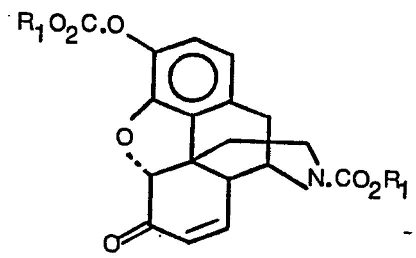

K

wherein R$_1$ is as defined above;

(C) acylating the 3-O,N-(di-R$_1$-oxycarbonyl) normorphinone having the formula (K) by reaction with an acylating agent selected from acid anhydrides of the formula $(R_2)_2O$ and acyl halides of the formula $R_2X'$, where R$_2$ is an acyl group and X' is a halogen atom, to form a 3-O,N-(di-R$_1$-oxycarbonyl) normorphinone dienol acylate having the formula (L):

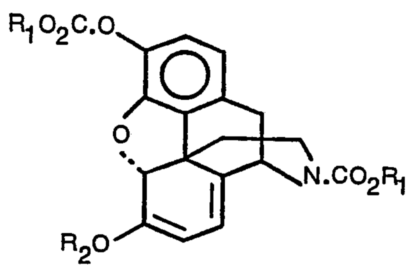

L

wherein R$_1$ and R$_2$ are as defined above;

(D) reacting the dienol acylate having the formula (L) with a peroxy oxidizing agent to form a 14-hydroxy-3-O,N-(di-R$_1$-oxycarbonyl) normorphinone having the formula (M)

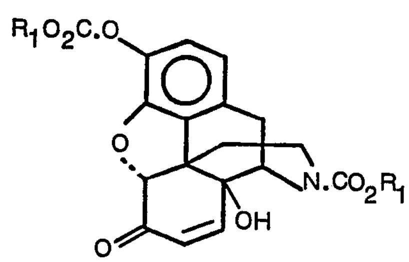

M

wherein R$_1$ is as defined above;

(E) reducing the 14-hydroxy-3-O,N-(di-R$_1$-oxycarbonyl) normorphinone having the formula (M) to form a 3-O,N-(di-R$_1$-oxycarbonyl) noroxymorphone haing the formula (N)

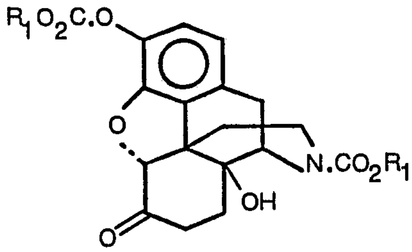

N

wherein $R_1$ is as defined above; and

(F) hydrolysing the 3-O,N-(di-$R_1$-oxycarbonyl) noroxymorphone having the formula (N) to form noroxymorphone having the formula (O)

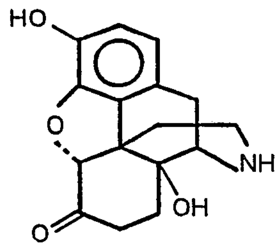

O

wherein $R_1$ is an alkyl, alkenyl, aralkyl or aryl group such that the $R_1$-oxycarbonyl groups attached to the 3-O and N atoms of the various intermediate compounds are alkyloxy, alkenyloxy, aralkyloxy or aryloxy groups.

The present invention also provides, as novel compositions of matter useful in the above process, the following compounds:

a 3-O,N-(di-$R_1$-oxycarbonyl normorphinone having the formula (K),

a 3-O,N-(di-$R_1$-oxycarbonyl normorphinone dienol acylate having the formula (L), and

a 14-hydroxy-3-O,N-(di-$R_1$-oxycarbonyl) normorphinone having the formula (M) wherein $R_1$ is as defined above.

In preferred embodiments of the above process and compounds, $R_1$ is ethyl and the dienol acylate is a denol acetate.

The overall process is illustrated below by the Reaction Scheme (shown on the next page of this application). In the formulae J, K, L, M and N shown in the Reaction Scheme, $R_1$ is as defined above and $R_2$ is an acyl group, with $R_1$ and $R_2$ preferably being an ethyl group and an acetyl group, respectively.

Steps A, B, C, D, E and F in the above process correspond to steps 1, 2, 3, 4, 5 and 6, respectively, of the Scheme.

The compounds prepared in such steps are illustrated by the formulae J, K, L, M, N and O, with formulae K, L and M corresponding respectively to the three above-listed novel compounds in the order in which such compounds are listed above.

(The Reaction Scheme is on the next page.)

Reaction Scheme

Morphine

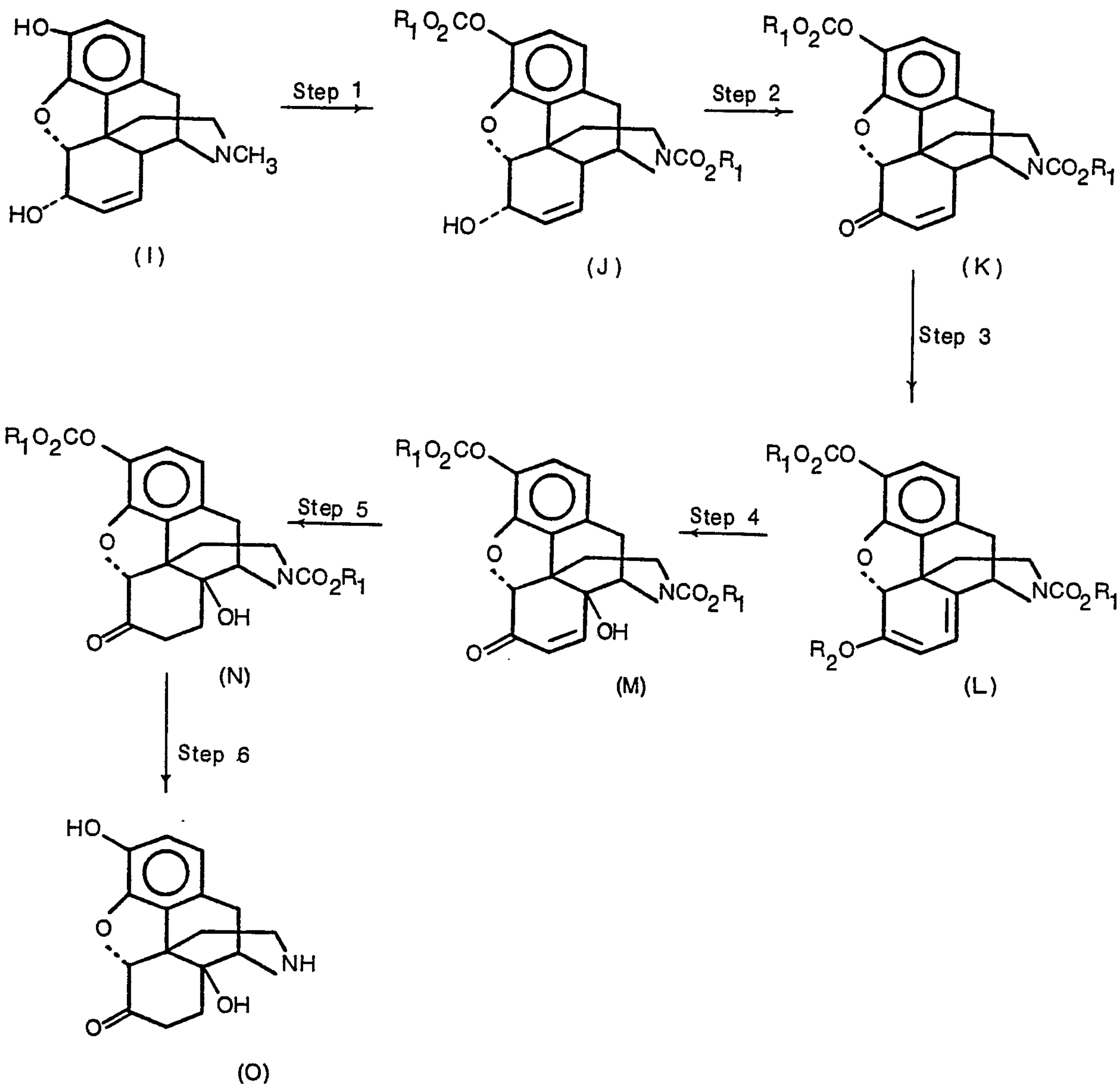


Noroxymorphone

In the process of the present invention for preparing noroxymorphone from morphine, the initial step (Step 1) is conversion of morphine to the compound of formula J by reaction of morphine with, for example, a haloformate ester of the formula X—C(=O)O$R_1$ where X is a halogen, preferably bromine or chlorine or more preferably chlorine (wherby the haloformate ester is a chloroformate), and $R_1$ is as defined above. Illustrative $R_1$ groups within the above definition are methyl, ethyl, propyl, heptyl, 1,1,1-trichlorethyl, butenyl, phenyl, benzyl, and naphthyl groups. Preferably $R_1$ is ethyl. Ethyl chloroformate is the preferred haloformate ester. The reaction is preferably carried out under an inert atmosphere in the presence of an inert organic liquid medium (hereinafter referred to as an "organic solvent") in which the reactants can be dissolved or dispersed under the reaction conditions employed to form a solution, dispersion, suspension or other reaction mixture. As used herein, the modifying term "inert" means that the substance referred to in connection with an associated reaction is at least substantially non-reactive with the reactants and desired product or products. The haloformate ester is preferably added slowly to a solution or other mixture of morphine in the organic solvent employed, with stirring.

The organic solvent may be, for example, an arene, ketone, ester, chlorinated alkane, chlorinated arene or a mixture of two or more of such organic solvents. Chloroform is preferred as the solvent.

The reaction is preferably promoted by the presence of a weak base which may be, for example, potassiumn bicarbonate, sodium bicarbonate, pyridine or substituted pyridine, triethylamine, imidazole, sodium carbonate, potassium carbonate or a mixture of two or more of such weak bases. Sodium bicarbonate is preferred. The weak base is preferably present in the mixture of morphine and organic solvent to which the haloformate ester is to be added.

Suitable conditions for the reaction in Step 1 include the addition or presence of, per mole of morphine: about 2—50 moles of a haloformate ester such as an alkyl, alkenyl, aralkyl, or aryl bromoformate or chloroformate (preferably about 7.8 moles of ethyl chloroformate), about 1—20 moles of weak base (preferably about 15 moles of sodium bicarbonate), and about 0.1—10 liters of organic solvent (preferably about 10 liters of chloroform). The reaction may suitably be effected at, for example, atmospheric pressure and a temperature of about 40—120°C, while about 90—100°C is preferred. The inert atmosphere is preferably nitrogen.

Preferably the reaction mixture is substantially anhydrous, i.e. the mixture does not contain more than 5% water and preferably not more than 1 to 2% water. Optimally, the reaction mixture is entirely free of water. Accordingly, the components used for preparing the reaction mixture are preferably at least substantially anhydrous. Water of hydration in morphine can be, and preferably is, removed by azeotropic distillation with toluene.

At the conclusion of the reaction, the product (i.e., the compound of Formula J) can be recovered by cooling the reaction mixture (preferably to about 25°C), and thereafter washing (e.g., by adding water at 25°C), drying and evaporating the solvent.

The balance of this description, including conditions and parameters for Steps 2—6 set forth below of the process for preparing noroxymorphone form morphine, is given with principal reference to such steps and associated compounds produced therein for the case where Step 1 is carried out using an ethylhaloformate ester (e.g., ethylchloroformate) and the acyl group ($R_2$) is acetyl by way of illustration. It is understood, however, that other $R_1$ and $R_2$ groups may be substituted in whole or in part for the ethyl and acetyl groups, respectively, in the balance of this description unless otherwise indicated.

Step 2

In the next step (Step 2) of the process for preparing noroxymorphone from morphine, oxidation of the compound of Formula J is effected by reactively contacting that compound with an oxidizing agent effective for oxidizing allylic OH groups (i.e., allylic hydroxyl groups) to keto groups, thereby preparing a 3-O,N-(di-$R_1$-oxycarbonyl)normorphinone of Formula K. Suitable oxidizing agents include chromium oxidants (e.g., chromium trioxide, pyridinium dichromate and t-butyl ammonium dichromate); benzophenone and cyclohexanone, each preferably employed with a base; manganese dioxide; and mixtures of dimethylsulfoxide with acetic anhydride. Chromium trioxide is preferred and advantageously may be employed in glacial acetic acid, in pyridine, in a mixture of pyridine and $CH_2Cl_2$, or in aqueous sulfuric acid. An especially preferred oxidizing agent is Jones Reagent (a solution of chromium trioxide in aqueous sulfuric acid).

The oxidation reaction is preferably carried out under an inert atmosphere in the presence of an inert organic liquid medium (hereinafter referred to as an "organic solvent") in which the reactants can be dissolved or dispersed under the reaction conditions employed to form a solution, dispersion, suspension or other reaction mixture. The organic solvent employed in the reaction of Step 2 is preferably acetone. The oxidizing agent is preferably added slowly to a solution or other mixture of the compound of Formula J in the organic solvent employed, with stirring.

Suitable conditions for the reaction in Step 2 include the addition or presence of, per mole of the compound of Formula J: about 0.7—6 (e.g., 0.7—6.0) moles of the oxidant or oxidizing agent (preferably about 1.1 moles of chromium trioxide as Jones Reagent) and about 0.1—10 liters of the organic solvent employed (preferably about 3.8 liters of acetone). The reaction may suitably be effected at, for example, atmospheric pressure and a temperature of about 0—25°C, while about 0—10°C is preferred. The inert atmosphere is preferably nitrogen. Anhydrous conditions are not required for Step 2. The product of Step 2, i.e., the compound of Formula K can be recovered in any suitable manner.

Where, as preferred, the oxidant is chromium trioxide in the form of Jones Reagent, the reaction advantageously is quenched after completion of the reaction using any suitable quenching procedure. Preferably, an alcohol and base are added to the reaction mixture to aid in quenching, followed by decanting the resulting mixture, washing the residual solids with acetone, decanting the resulting decanted mixtures or solutions and evaporating at least a substantial portion of the acetone and alcohol from the combination to give a compound of Formula K in the form of an oil, which preferably is crystallized from 2B anhydrous ethanol to improve the purity of the product. Isopropyl alcohol is preferably employed as the quenching alcohol, while sodium bicarbonate is preferred for the quenching base. These compounds have been found to be highly effective for neutralizing the reaction mixture containing Jones Reagent and facilitating recovery of larger amounts of higher purity compound K.

If desired, recovery of compound K from the reaction mixture may include a step of extraction of the quenched reaction mixture. Evaporation of the acetone solution should precede extraction. Such extraction advantageously is effected using an organic solvent which may be, for example, an arene, ketone, ester, chlorinated alkane or chlorinated arene capable of preferentially dissolving compound K and thereafter

evaporating the extract solvent to yield an oil which can be crystallized as above to yield compound K in crystalline form.

The compounds of Formula K are novel intermediates useful in the synthesis of noroxymorphone in accordance with this invention.

Step 3

In the next step (Step 3) of the process for preparing noroxymorphone from morphine, acylation of the compound of Formula K is effected by reactively contacting that compound with an acylating agent which is an acid anhydride of the formula $(R_2)_2O$ or an acyl halide of the formula $R_2X'$ where $R_2$ is as defined above and X' is halogen, whereby the dienol acylate compound of Formula L is prepared. Preferably, X' is bromide or more preferably chloride, and R is acetyl.

The acetylation or other acylation reaction is preferably carried out under an inert atmosphere in the presence of a catalytic acid (hereinafter referred to as an "acid catalyst") or more preferably in the presence of a catalytic base (hereinafter referred to as a "base catalyst").

Suitable conditions for the reaction in Step 3 include the addition or presence of, per mole of the compound of Formula K: about 0.091—5 (e.g., 0.01—5.0) moles of base (i.e., base catalyst) and about 1—100 moles of acylating agent. The reaction may suitably be effected at, for example, atmospheric pressure and a temperature of about 25—140°C, while about 100—105°C is preferred. The inert atmosphere is preferably nitrogen. Anhydrous conditions substantially as described above for Step 1 are preferred.

Suitable acylating agents include, for example, acetyl chloride and mixed anhydrides of acetic acid. Acetic anhydride is preferred. The base catalyst may be, for example, sodium or potassium acetate, pyridine, triethylamine or mixtures of 2 or more of the foregoing bases. Sodium acetate is generally preferred as the catalyst, especially where the preferred acylating agent (acetic anhydride) is employed. Suitable acid catalysts include, for example, p-toluene sulfonic acid and boron trifluoride etherate. The conditions and parameters set forth above for Step 3 are generally applicable for use with the various $R_1$ and $R_2$ groups within their above definitions are employed.

Preferably, per mole of a compound of Formula K, there are employed about 1.0 moles of preferably anhydrous sodium acetate and about 19.8 moles of acetic anhyride, also used as solvent so a large molar excess is preferred.

At the conclusion of the reaction, which may be completed within, for example, about two hours, the 3,17-(di-$R_1$-oxycarbonyl)normorphinone enol acylate of Formula L (e.g., 3,17-(diethoxycarbonyl)-normorphinone enol acetate) may be recovered from the reaction mixture in any suitable manner. Advantaeously, following completion of the reaction, the reaction mixture is cooled (preferably to about 25°C), and recovery is effected employing an extraction procedure. Such extraction advantageously is effected using an organic solvent which may be, for example, an arene, ketone, ester, chlorinated alkane or chlorinated arene capable of preferentially dissolving Compound L and thereafter evaporating the extract solvent to yield an oil (typically dark brown). Chloroform is preferred for use as the extraction solvent. Preferably, the solvent extracted reaction mixture is washed with water, the resulting organic layer is dried by stirring over anhydrous sodium sulfate, followed by evaporation of the solvent to yield the oil, i.e., the desired compound L.

Step 4

In the next step (Step 4) of the process for preparing noroxymorphone form morphine, the 14-hydroxy-3-O,N-disubstituted normorphinone compound of Formula M [i.e., 14-hydroxy-3-O,N-(di-$R_1$-oxycarbonyl) normorphinone] is prepared by reactively contacting the compound of Formula L with a peroxy oxidation agent capable of introducing a B-oriented (i.e., beta-oriented) —OH group in the 14- position of the dienol acetate or other dienol acylate of Formula L. Such peroxy oxidation agent may be, for example, an aromatic or aliphatic, mono-basic or poly-basic carboxylic peroxy-acid. Suitable peroxy-acids include, for example, substituted or unsubstituted perbnezoic acid wherein the substituent may be, for example, chloro, bromo, iodo, fluoro or nitro; monoperphthalic acid; performic acid; peracetic acid; monopermaleic acid; trifluoroperacetic acid; and trichloroperacetic acid. In chloro-, bromo-, iodo- and fluoroperbenzoic acids, the indicated halogen substituent is preferably in the meta position. The nitro group in nitroperbenzoic acid is preferably in the para position. The peroxy-acid is preferably chloroperbenzoic acid and more preferably is m-chloroperbenzoic acid.

It is understood that the term "3-O,N-" employed herein as a prefix in naming various compounds can be replaced by the term "3, 17-".

The peroxy-acid may be formed in situ, that is, in the presence of the dienol acylate, by reaction of hydrogen peroxide with the corresponding acid or corresponding acid anhydride. Preferably, however, contacting of the dienol acylate is effected with peroxy-acid prepared outside the presence thereof.

The act of reactively contacting the dienol acylate of Formula L with the peroxy oxidation agent is effected under Reaction Conditions effective for introducing or substituting an —OH group in the 14-position of the dienol acylate such that the compound of Formula M is prepared.

Preferably, the peroxy oxidation agent, sometimes referred to herein simply as the peroxy-acid or per-acid, is added to a solution or other mixture containing the dienol-acyalte in an inert organic solvent. That is, the organic solvent is a solvent for and substantially non-reactive with the 3-O,N-(di-$R_1$-

oxycarbonyl)normorphinone enol acylate and the per-acid. The solvent advantageously is present in a solubilizing amount for each of the dienol acylate and the per-acid.

The organic solvent is preferably a polar organic solvent. Suitable classes of solvents include carboxylic acids, aprotic polar solvents, chlorinated hydrocarbons, carboxylic acid nitriles, carboxylic acid esters, ethers, mixtures thereof and the like. Carboxylic acids, aprotic polar solvents, chlorinated hydrocarbons and mixtures thereof are generally preferred. Suitable solvents include, for example, acetic acid, dimethylformamide, chloroform, methylene chloride (dichloromethane), acetonitrile, 1,2-dimethoxymethane, propyl acetate and mixtures thereof. Acetic acid is preferred, while glacial acetic acid is most preferred.

In addition to the solvent and the dienol acylate, the reaction mixture may include other components. For example, the reaction mixture may include agents effective for inhibiting formation of 7,8-epoxide derivatives of the dienol acylate and other side reaction products.

The reaction mixture preferably further incudes, as an acid catalyst, an acid having a $pK_a$ of from about 0 to about 3 (e.g., to about 3.0 or more). It has surprisingly been found that inclusion of such acid permits preparation of the 14-hydroxy compound of Formula M in higher yield. Suitable acids include, for example, oxalic acid, trichloracetic acid, trifluoroacetic acid, and methanesulfonic acid, as well as phosphoric acid, chloroacetic acid, maleic acid, and mixtures of two or more of such acids. Oxalic acid is preferred.

The reaction conditions preferably further include effecting the contacting of the dienol acylate with the per-acid in the substantial absence of water, i.e., with water not present in an amount greater than 5 percent by weight based on the weight of the reaction mixture. Water is preferably not present in an amount more than 2 percent, and more preferably not present in an amount more than 0.5 percent, on the same basis. Anhydrous reaction conditions are most preferred. Such non-aqueous or anhydrous conditions result in higher yields than are generally attainable under aqueous conditions. Anhydrous reaction conditions may conveniently be provided by employing anhydrous components for the reaction mixture and conducting the reaction under an inert anhydrous atmosphere, e.g., dry nitrogen. Preferably, substantially no water of hydration is present in any of the components of the reaction mixture. Thus, for example, oxalic acid dihydrate may be converted to anhydrous oxalic acid by heating the dihydrate four hours at 100—110°, followed by cooling in the presence of a dessicant in a dessicator or othe air-tight container. Similarly, commercially available peracetic acid (usually containing 10—15% water) advantageously is dried, as by drying with $Na_2SO_4$ (e.g., 20 g per 100 ml) for several hours, decanting and drying at least 16 hours with 4A molecular sieves (e.g., 20 g per 100 ml). The per-acid is preferably added as a solid. Normally liquid peracids (e.g., peracetic acid and performic acid are preferably added as solutions thereof in an inert polar solvent, which may be, for example, methylene chloride or the parent or corresponding acid (i.e., acetic acid and formic acid for peracetic acid and performic acid, respectively).

The per-acid is preferably added incrementally to the dienol acylate reaction mixture. The addition may advantageously be made in discrete portions, and at an average rate of from about 0.01 to about 0.1 gram-equivalents per minute per mole of the dienol acylate. Desirably, a total of at least 1 gram-equivalent of the per-acid is added per mole of the dienol acylate. The addition may advantageously be made over a period from about 30 to about 120 minutes, preferably with stirring of the reaction mixture.

The reaction may effectively be conducted at any suitable pressure (preferably about atmospheric pressure) and at any suitable temperature, e.g., about 10—100°C (i.e., from about 10°C to about 100°C), preferably about 15—25°C (i.e., from about 15°C to about 25°C). The peroxy-acid may be added in a total amount of, for example, about 1—2.5 moles (i.e., from about 1 to about 2.5 moles) per mole of the dienol acylate (pure basis), preferably from about 1.1 to about 1.4 moles and more preferably about 1.4 moles on the same basis. The amount of peroxy-acid is preferably increased where the crude dienol acylate starting material is of low assay. The catalyst acid may be employed in an amount of, for example, about 0.01-0.5 moles (i.e., from about 0.01 to about 0.5 mole), preferably about 0.5 mole, per mole of the dienol acylate. The solvent may be present in an amount of, for example, about 0.5—10 liters (i.e., from about 0.5 to about 10 liters), preferably about 2.5 liters per mole of the dienol acetate.

Where an acid catalyst is included in the reaction mixture, preferably the solvent is additionally a solvent for, and substantially non-reactive with, the acid catalyst and present in a solubilizing amount therefor.

At the completion of the reaction, the 14-hydroxy compound of Formula M can conveniently be recovered. Recovery can be effected readily by quenching the reaction mixture with water or preferably an aqueous alkali (e.g., an aqueous solution of $NH_4OH$, NaOH, KOH, $NaHCO_3$, $NaH_2PO_4$, $Na_2HPO_4$ or a mixture of such alkalis). An aqueous mixture of NaOH and $NH_4OH$ is preferred. Thereafter, the 14-hydroxy compound can be separated by filtration of the quenched mixture, followed by extraction of the filtrate with a water-immiscible organic solvent, which may be, for example, an arene, ketone, ester, alcohol, chlorinated alkane, chlorinated arene, or a mixture of such solvents, provided that the solvent or mixture is capable of preferentially dissolving the compound of Formula M, and preferably is chloroform; evaporation of the extraction solvent; and drying of the resulting solid.

Step 5

In the next step (Step 5) of the process for preparing noroxymorphone form morphone, [for example, preparing 3-O,N-(diethoxycarbonyl)noroxymorphone from 3-O,N-(diethoxycarbonyl)-14-hydroxynormorphinone], reduction of the compound of Formula M is effected, preferably by catalytic hydrogenation of that compound, thereby preparing a 3-O,N-(di-$R_1$-oxycarbonyl)noroxymorphone of Formula N. Suitable catalysts include, for example, noble metal catalysts, which may be supported on a suitable support and may be chemically combined (e.g., platinum on carbon, palladium on carbon, rhodium on carbon, and platinum oxide). Charcoal-supported 5% palladium is preferred (in an amount sufficient to provide about 0.1 part palladium per part of compound M).

The reduction reaction is preferably carried out in the presence of an inert organic liquid medium (hereinafter referred to as an "organic solvent") in which compound M can be dissolved or dispersed under the reaction conditions employed to form a solution, dispersion, suspension or other reaction mixture. The organic solvent employed in the reaction of Step 5 may be, for example, an alcohol (e.g., ethanol), an ester (e.g., ethyl acetate), or an acid (e.g., acetic acid or formic acid). The solvent is preferably glacial acetic acid.

Suitable conditions for the reaction in Sep 5 include the addition or presence of, per mole of the compuond of Formula M: about 0.1—100 g (grams) of the catalyst employed (preferably about 45 grams of 5% Pd on charcoal) and about 0.1—10 liters of the solvent employed (preferably about 3.9 liters of glacial acetic acid). The reaction may suitably be effected at, for example, about 1—10 atmospheres pressure or more (preferably about 3 atmospheres) and a temperature of about 25—80°C, while about 25—40°C is preferred (more preferably about 40°C). The reaction can be completed within about 3 hours under the preferred conditions set forth above.

The product of Step 5, i.e. the compound of Formula N, can be recovered in any suitable manner. Recovery is preferably effected by filtering the reaction mixture through Celite diatomaceous earth to remove the catalyst; evaporating solvent from the filtrate; dissolving the filtration residue in a mixture of 80% chloroform and 20% toluene, washing the resulting solution with water, adding to the resulting organic layer a sufficient amount of alkali (e.g., aqueous NaOH) with stirring and cooling to obtain a pH of about 8.5, removing the resulting aqueous layer, washing the residual organic phase with water, removing the washed organic phase and evaporating solvent therefrom; and combining the portions of the product thus recovered from the filtrate and from the filtration residue.

Step 6

In the next step (Step 6) of the process for preparing noroxymorphone from morphine [for example, preparing noroxymorphone from 3-O,N-(diethoxycabonyl)noroxymorphone], hydrolysis of the compound of Formula N is effected, preferably by contacting that compound with an acidic or basic hydrolysis catalyst in the presence preferably of water under hydrolysis conditions, thereby preparing the corresponding hydrolysis-catalyst salt of noroxymorphone. Thereafter, noroxymorphone is recovered by preferably neutralizing the hydrolysis mixture with a neutralizing agent, filtering the neutralized mixture, and washing and drying the filtrate. Suitable acidic hydrolysis catalysts include, for example, methane sulfonic acid, p-toluene sulfonic acid, trichloroacetic acid, hydrobromic acid (preferably in glacial acetic acid), hydrochloric acid (preferably in glacial acetic acid), mixtures of formic acid and strong acid (sometimes referred to as formic acid/strong acid), 20% hydrochloric acid, and mixtures of n-butanol and strong acid (sometimes referred to as b-butanol/strong acid), and sulfuric acid. Suitable basic hydrolysis catlaysts include, for example, potassium hydroxide, which may be employed as a solution thereof in ethanol, water, or diethylene glycol or the like. Sulfuric acid is preferred. Where sulfuric acid is employed as the hydrolysis catalyst, the neutralizing agent is preferably aqueous ammonium hydroxide.

The hydrolysis is preferably carried out under an inert atmosphere in the presence of 8N aqueous sulfuric acid.

Suitable conditions for the hydrolysis reaction in Step 6 include the addition of an amount of sulfuric acid (aqueous) corresponding to about 0.1—10 liters of 8N aqueous sulfuric acid per mole of the 3,17-(di-$R_1$-oxycarbonyl) noroxymorphone compound employed (preferably abot 2 liters of 8N aqueous sulfuric acid on the same basis). Hydrolysis may suitably be effected at, for example, atmospheric pressure and a temperature of about 90—100°C, while about 100—105°C is preferred. The inert atmosphere is preferably nitrogen.

Although it is advantageous to cool the reaction mixture following completion of the reaction in Step 4 (as described above) and before the extraction procedure, such cooling can satisfactorily be omitted. Similarly, recovery of the 14-hydroxy compound of formula M can satisfactorily be omitted prior to hydrogenation thereof. If such recovery is omitted, the compound of formula M can be hydrogenated directly in situ in the reaction mixture after completion of the reaction in Step 4. Where the compound of formula N is prepared by such in situ method, afte the recovery procedure including filtration and solvent evaporation as set forth above for Step 5, the recovery procedure set forth for Step 4 involving use of an aqueous mixture of NaOH and $NH_4OH$ and filtration is preferably employed to aid in removing the acid corresponding to the per-acid employed (e.g. m-chlorobenzoic acid removal where m-chloroperbenzoic acid is the per-acid) and the acid catalyst employed in Step 4. The extraction set forth for Step 4 is also preferably employed in accordance with the above description thereof for this in-situ embodiment.

EXAMPLES

Practice of the present invention is illustrated by the following examples. All parts and percentages given throughout this disclosure are by weight unless otherwise indicated. Unless otherwise indicated, the

identity of the compounds prepared in each example was confirmed by mass spectroscopy, IR and NMR (proton and carbon-13) and the reaction pressure in each example was approximately atmospheric.

### Example 1

Preparation of 3-O,N-(diethoxycarbonyl)normorphine from morphine (Step 1)

A suspension of 60.6 g morphine·$H_2O$ in 600 ml toluene was refluxed under Dean-Stark conditions for 3 hours under nitrogen. Then the toluene was stripped under vacuum and 300 g potassium bicarbonate and 2000 ml chloroform were added. To the stirred mixture, 149 ml ethyl chloroformate was added slowly over 5 minutes. The resulting solution was refluxed for 6 hours under nitrogen; after cooling to 25°C, 1000 ml water was added to the stirred mixture, resulting in formation of an organic phase containing the product and an aqueous phase containing water-miscible solids which were removed from the reaction mixture by the addition. The aqueous layer was removed and the organic phase was washed once with 200 ml water, dried over anhydrous sodium sulfate and concentrated. The solid obtained was recrystallised from ethyl acetate/hexane to give 78 g 3-O,N-(diethoxycarbonyl) normorphine, corresponding to 94% yield.

### Example 2

Preparation of 3-O,N-(diethoxycarbonyl)normorphine from 3-O,N-(diethoxycarbonyl) normorphine (Step 2)

A solution of 50.1 g 3-O,N-(diethoxycarbonyl) normorphine (prepared substantially as set forth in Example 1), in 450 ml acetone was cooled to 0—10°C under nitrogen. Then 46 ml of a Jones reagent solution (containing 13.2 g chromium trioxide, 12 ml concentrated sulfuric acid and 55 ml water) was added dropwise, while maintaining the temperature at 0—10°C. At the end of the Jones reagent addition, 20 ml isopropyl alcohol and 50 g solid sodium bicarbonate were added and the mixture was stirred 30 minutes at 25°C. Next, the acetone solution was decanted and the residual solids were washed two times with 50 ml acetone and decanted. The decanted acetone solutions were combined and evaporated to give a yellow oil which crystallized from 2B anhydrous ethanol to give 33.5 g 3-O,N-(diethoxycarbonyl) normorphinone, which corresponds to 81% yield.

### Example 3

Preparation of 3-O,N-(diethoxycarbonyl) normorphinone dienol acetate from 3-O,N-(diethoxycarbonyl) normorphinone (Step 3)

A mixture of 94 g 3-O,N-(diethoxycarbonyl) normorphinone (prepared substantially as set forth in Example 2), 18.7 g anhydrous sodium acetate and 430 ml acetic anhydride was heated at 100—105°C under nitrogen for 2 hours. The solution was cooled to 25°C, 500 ml chloroform were added and the organic mixture was washed two times with 100 ml water. The organic layer was dried thoroughly by stirring over anhydrous sodium sulfate for 30 minutes. Concentration of the resulting solution gave a dark brown oil assaying for 93 g of 3-O,N-(diethoxycarbonyl) normorphinone dienol acetate, which corresponds to 90% yield.

### Example 4

Preparation of 3-O,N-(diethoxycarbonyl)-14-hydroxynormorphinone from 3-O,N-(diethoxycarbonyl) normorphinone dienol acetate (Step 4)

Crude 3-O,N-(diethoxycarbonyl) normorphinone dienol acetate (approximately 90 to 95% purity and prepared substantially as set forth in Example 3), containing 104 g pure compound, and 9.5 g anhydrous oxalic acid were dissolved in 575 ml glacial acetic acid and cooled to 15°C. Then a total of 54.5 g m-chloroperbenzoic acid was added in approximately equal portions at 10 minute intervals with stirring. After about 10—15 minutes following the final addition, the reaction mixture was poured into a mixture of 20 ml 50% NaOH, 800 ml $NH_4OH$ and 800 g ice with stirring and the product was extracted with chloroform several times. The combined chloroform extracts were concentrated to give a residue assaying for 88g 3-O,N-(diethoxycarbonyl)-14-hydroxy normorphinone, which corresponds to 90% yield.

### Example 5

Preparation of 3-O,N-(diethoxycarbonyl) noroxymorphone from 3-O,N-(diethoxycarbonyl)-14-hydroxy normorphinone (Step 5)

A solution of 49 g 3-O,N-(diethoxycarbonyl)-14-hydroxy normorphinone (prepared substantially as set forth in Example 4) in 425 ml glacial acetic acid was added carefully to a hydrogenation bottle containing 5 g of 5% Pd on charcoal in 10 ml glacial acetic acid. The resulting mixture was shaken in a Parr apparatus under hydrogen atmosphere at 40°C and a substantially constant reaction chamber pressure of about 40 psig for 3 hours. After the hydrogenation reaction was substantially complete as indicated by HPLC monitoring of the reaction, the reaction mixture was cooled to about 25°C, the hydrogen supply was shut off and the reaction chamber was flushed with $N_2$. Thereafter the catalyst was removed by filtration through Celite diatomaceous earth and the filtrate was concentrated. The residue was dissovled in 4:1 chloroform/toluene and washed two times with 50 ml water. The organic layer was stirred and cooled while adding 1 *N* NaOH solution until pH of 8.5 was reached. The aqueous layer was drawn off and the organic phase was washed two times with 100 ml water. Evaporation of solvents from the washed organic phase gave 49 g of 3,17-(diethyoxycarbonyl); noroxymorphone assaying for 100% yield by liquid chromatography.

Example 6

Preparation of noroxymorphone from 3-O,N-(diethoxycarbonyl) noroxymorphone (Step 6)

Crude 3,17-(diethoxycarbonyl) noroxymorphone (approximately 90 to 98% purity and prepared substantially as set forth in Example 5) containing 28 g pure compound was heated in contact with 128 ml 8*N* sulfuric acid solution at 100—105°C and the sulfate salt of crude noroxymorphone was collected. The salt was dissolved in water and concentrated HCl was added to adjust the pH to 4.5; 2 g charcoal and 2 g filter aid was added and the resulting mixture was heated at 50°C for 10 minutes. The hot mixture was filtered and after cooling to 25°C, the filtrate was adjusted to pH 8.8—9.0 with concentrated ammonium hydroxide, thereby precipitating noroxymorphone (a solid). The solid was filtered, washed with water and dried 3 hr at 90°C to give 12.5 g noroxymorphone at 90% assay, corresponding to 60% yield for Step 6 in the overall synthesis of noroxymorphone.

The overall yield of noroxymorphone for the six steps of Examples 1—6 is 37% based on morphine.

In a large number of runs starting with 50 to 120 grams morphine, typical yields were 94—96% for Step 1 and 81—87% for Step 2.

Example 7

Preparation of Noroxymorphone from 3-O,N-(diethoxycarbonyl) normorphinone

Step 3

A mixture of 26.9 g 3-O,N-(diethoxycarbonyl) normorphinone (prepared in accordance with the procedure of Example 2), 5.3 g anhydroux sodium acetate and 122 ml acetic anhydride was heated at 100—105°C under nitrogen for 2 hours. The solution was cooled to 25°C, 140 ml chloroform were added and the organic mixture was washed two times with 30 ml water. The organic layer was dried thoroughly by stirring over anhydrous sodium sulfate for 30 minutes. Concentration of the resulting solution gave a dark brown oil assaying for 26.5 g of 3-O,N-(diethoxycarbonyl) normorphinone dienol aceate, which corresponds to 90% yield.

Step 4

The dienol acetate oil and 2.7 g anhydrous oxalic acid were dissolved in 180 ml glacial acetic acid by adding the glacial acetic acid to the oil with stirring, followed by adding the oxalic acid with stirring. The resulting solution was cooled to 15°C. Then a total of 15.4 g -chloroperbenzoic acid was added in approximately equal portions at 10 minute intervals with stirring. After about 10—15 minutes following the final addition, about 6 ml water was added and the resulting solution was stirred for about 30 minutes. At the end of such time, the solution assayed for 20.9 g 3-O,N-(diethoxycarbonyl)-14-hydroxy normorphinone, which corresponds to 84% yield for Step 4.

Step 5

To the solution of 20.9 g 3-O,N-(diethoxycarbonyl) 14-hydroxynormorphinone prepared in Step 4 was added sufficient glacial acetic acid, with stirring, to increase the volume of the solution to 230 ml. The resulting solution was added carefully to a hydrogenation bottle containing 27 g of 5% Pd on charcoal in 10 ml glacial acetic acid. The resulting mixture was shaken in a Parr apparatus under hydrogen atmosphere at 40°C for 3 hours. The catalyst was removed by filtration through Celite diatomaceous earth and the filtrate was concentrated. The residue was dissolved in 4:1 chloroform/toluene and washed two times with 50 ml water. The organic layer was stirred and cooled while adding 1*N* NaOH solution until pH of 8.5 was reached. The aqueous layer was drawn off and the organic phase was washed two times with 50 ml water. Evaporation of solvents gave 20.0 g of 3,17-(diethoxycarbonyl) noroxymorphone assaying for 95% yield for Step 5 by liquid chromatography.

Step 6

Crude 3,17-(diethoxycarbonyl) noroxymorphone containing 20.0 g pure compound was heated with 128 ml 8*N* sulfuric acid solution at 100—105°C under nitrogen for 12 hours. The solution was cooled to 25°C and the sulfate salt of crude noroxymorphone was collected. The salt was dissolved in water and concentrated HCl was added to adjust the pH to 4.5; 2 g charcoal and 2 g filter aid was added and heated at 50°C for 10 minutes. The hot solution was filtered and after cooling to 25°C, the filtrate was adjusted to pH 8.8—9.0 with concentrated ammonium hydroxide. The solid was filtered, washed with water and dried 3 hr at 90°C to give 11.1 g noroxymorphone, corresponding to 84% yield for Step 6.

The overall yield of noroxymorphone for the four steps of Example 7 is 60% based on the substituted morphine starting material. Taken with the typical yields set forth above for Steps 1 and 2, it is seen that noroxymorphone can be readily prepared from morphine in 46—50% or more overall yield based on morphine.

The following Examples illustrate use of the indicated peroxy-acid compounds for carrying out Step 4.

Example 8

To *2.0 g powdered maleic* anhydride in *8* ml methylene chloride cooled to 10° under nitrogen was added *0.5* g 67% hydrogen peroxide. The mixture was stirred at 10° for 15 min. Then a solution of *4.4*g 3,17-O,N-(diethoxycarbonyl)normorphinone dienol acetate in *12* ml methylene chloride was added dropwise. The

resulting solution was stirred at 10° for *1.5* hours. Then 0.5 ml water was added, followed by vigorous stirring for 15 minutes. The layers were allowed to separate and the aqueous phase was extracted two times with 25 ml methylene chloride. The combined organic layers were washed successively with 20 ml dilute sodium bicarbonate solution and 10 ml water. Then the organic layers were dried over anhydrous sodium sulfate and evaporated to give *3.5* g 14-hydroxy-3,17-O,N-(diethoxycarbonyl) normorphinone which corresponds to *85*% yield.

Example 9

To 1.3 ml trifluoroacetic anhydride in *4* ml methylene chloride cooled to 10° under nitrogen was added *0.25* g 67% hydrogen peroxide. The mixture was stirred at 10° for 15 min. Then a solution of *22* g 3,17-O,N-(diethoxycarbonylnormorphinone dienol acetatete in *4* ml methylene chloride* was added dropwise. The resulting solution was stirred at 10° for *2* hours. Then 0.5 ml water was added, followed by vigorous stirring for 15 minutes. The layers were allowed to separate and the aqueous phase was extracted two times with 25 ml methylene chloride. The combined organic layers were washed successively with 20 ml dilute sodium bicarbonate solution and 10 ml water. Then the organic layers were dried over anhydrous sodium sulfate and evaporated to give *0.88* g 14-hydroxy-3,17-O,N-(diethboxycarbonyl)normorphinone which corresponds to *43*% yield.

Example 10

A solution of *2.2* g 3,17-O,N-(diethoxycarbonyl)normorphinone dienol acetate in *5* ml of *glacial acetic acid* was cooled to 20° under nitrogen. Then *0.25* g of 67% hydrogen peroxide was added dropwise and the solution was stirred at *90*° for *2* hrs. Then 5 ml water was added and the solution was extracted two times with 50 ml methylene chloride. The organic solution was washed with dilute sodium bicarbonate solution, dried over anhydrous sodium sulfate, and evaporated to give *0.78* g 14-hydroxy-3,17-O,N-(diethoxycarbonyl)normorphinone (*38*% yield).

Example 11

A solution of *2.2* g 3,17-O,N-(diethoxycarbonyl)normorphinone dienol acetate in *4* ml of *88% formic acid* was cooled to 20° under nitrogen then *0.25* g of 67% hydrogen peroxide was added dropwise and the solution was stirred at *25*° for *1* hr. Then 5 ml water was added and the solution was extracted two times with 50 ml methylene chloride. The organic solution was washed with dilute sodium bicarbonate solution, dried over anhydrous sodium sulfate, and evaporated to give *0.68* g 14-hydroxy-3,17-O,N-(diethoxycarbonyl)normorphinone (*33*% yield).

Noroxymorphone can be converted to antagonists for narcotics and other therapeutically useful products such as naloxone, naltrexone and nalbuphine. See U.S. Patents 3,254,088 (naloxone); 3,332,950 (naltrexone); and 3,393,197 (nalbuphine).

BEST MODE CONTEMPLATED

The best mode contemplated for carrying out this invention has been set forth in the above description, for example, by way of setting forth preferred materials and operating conditions, including preferred ranges and values of amounts and other nonobvious variables material to successfully practicing the invention in the best way contemplated at the time of executing this patent application.

**Claims**

1. A process for preparing noroxymorphone from morphine which comprises:
(A) converting morphine having the formula (I)

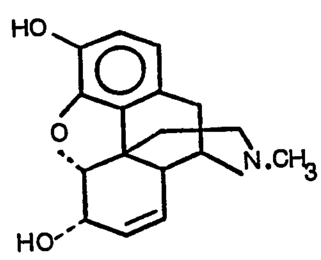

I

by reaction with a haloformate ester of the formula X—C(=O)$OR_1$, wherein $R_1$ is a $C_1$ to $C_7$ alkyl, 1,1,1-trichlorethyl, butenyl, benzyl, phenyl or naphthyl group and X is a halogen atom, to a 3-O,N-(di-$R_1$-oxycarbonyl) normorphine having the formula (J)

* note: The same yield is obtained if glacial acetic acid is used.

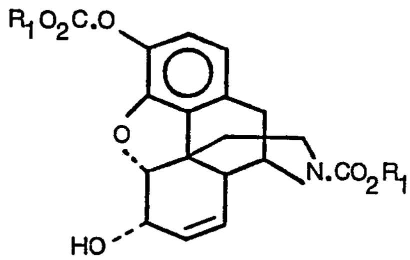

J

wherein $R_1$ is as defined above;

(B) oxidizing the 3-O,N-(di-$R_1$-oxycarbonyl) normorphine having the formula (J) by reaction with an oxidizing agent effective for oxidizing allylic —OH groups to keto groups to form a 3-O,N-(di-$R_1$-oxycarbonyl)-normorphinone having the formula (K)

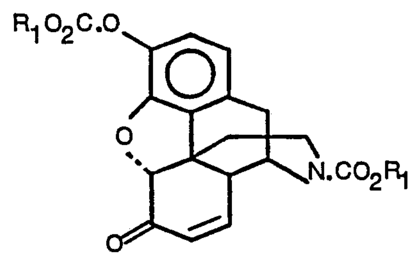

K

wherein $R_1$ is as defined above;

(C) acylating the 3-O,N-(di-$R_1$-oxycarbonyl) normorphinone having the formula (K) by reaction with an acylating agent selected from acid anhydrides of the formula $(R_2)_2O$ and acyl halides of the formula $R_2X'$, where $R_2$ is an acyl group and X' is a halogen atom, to form a 3-O,N-(di-$R_1$-oxycarbonyl)normorphinone dienol acylate having the formula (L):

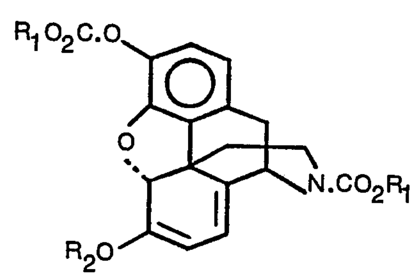

L

wherein $R_1$ and $R_2$ are as defined above;

(D) reacting the dienol acylate having the formula (L) with a peroxy oxidizing agent to form a 14-hydroxy-3-O,N-(di-$R_1$-oxycarbonyl) normorphinone having the formula (M)

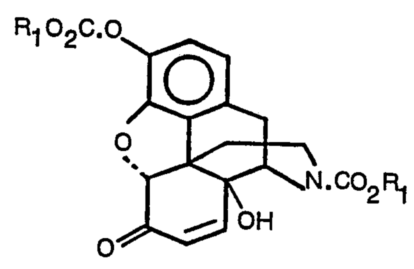

M

wherein $R_1$ is as defined above;

(E) reducing the 14-hydroxy-3-O,N-(di-$R_1$-oxycarbonyl) normorphinone having the formula (M) to form a 3-O,N-(di-$R_1$-oxycarbonyl) noroxymorphone haing the formula (N)

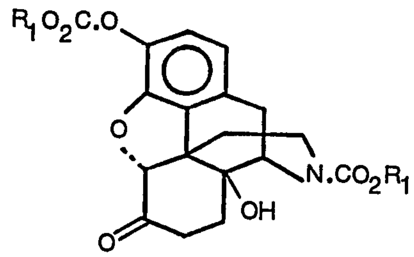

N

wherein $R_1$ is as defined above; and

(F) hydrolysing the 3-O,N-(di-$R_1$-oxycarbonyl) noroxymorphone having the formula (N) to form noroxymorphone having the formula (O)

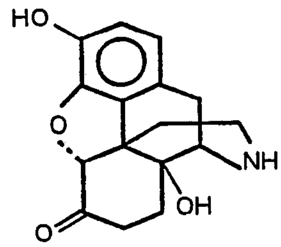

O

2. A process according to claim 1, in which $R_1$ is methyl, ethyl, propyl, heptyl, 1,1,1-trichlorethyl, vinyl, butenyl, phenyl, benzyl, or naphthyl.

3. A process according to claim 1 or claim 2, in which $R_1$ is ethyl.

4. A process according to any one of claims 1 to 3, in which $R_2$ is acetyl and in which the 3-O,N-(di-$R_1$-oxycarbonyl) normorphinone dienol acylate having the formula (L) is a 3-O,N-(di-$R_1$-oxycarbonyl) normorphinone dienol acetate.

5. A 3-O,N-(di-$R_1$-oxycarbonyl) normorphinone having the formula (K) set out in claim 1.

6. A compound according to claim 5, in which $R_1$ in formula (K) is ethyl whereby said compound is 3-O,N-(diethoxycarbonyl) normorphinone.

7. A 3-O,N-(di-$R_1$-oxycarbonyl) normorphinone dienol acylate having the formula (L) set out in claim 1.

8. A compound according to claim 7, in which $R_2$ in formula (L) is acetyl whereby said compound is a 3-O,N-(di-$R_1$-oxycarbonyl) normorphinone enol acetate.

9. A compound according to claim 8, in which $R_1$ in formula (L) is ethyl whereby said compound is 3-O,N-(diethoxycarbonyl) normorphinone enol acetate.

10. A 14-hydroxy-3-O,N-(di-$R_1$-oxycarbonyl) normorphinone having the formula (M) set out in claim 1.

11. A compound according to claim 10, in which $R_1$ in formula (M) is ethyl whereby said compound is 14-hydroxy-3-O,N-(diethoxycarbonyl) normorphinone.

12. A process for preparing a 3-O,N-(di-$R_1$-oxycarbonyl) normorphinone having the formula (K) set out in claim 1, which comprises reactively contacting a 3-O,N-(di-$R_1$-oxycarbonyl) normorphinone haing the formula (J) set out in claim 1 with an oxidizing agent effective for oxidizing allylic —OH groups to keto groups, whereby a compound having the formula (K) set out in claim 1 is prepared.

13. A process according to claim 12, in which the oxidizing agent is chromium trioxide.

14. A process for preparing 3-O,N-(di-$R_1$-oxycarbonyl) normorphinone dienol acylate having the formula (L) set out in claim 1, which comprises reactively contacting a compound of the formula (K) set out in claim 1 with an acylating agent which is an acid anhydride having the formula $(R_2)_2O$ or an acyl halide having the formula $R_2X'$, wherein $R_2$ is an acyl group and X' is a halogen atom, whereby a 3-O,N-(di-$R_1$-oxycarbonyl) normorphinone dienol acylate having the formula (L) set out in claim 1 is prepared.

15. A process according to claim 14, in which said acylating agent is acetic anhydride and the contacting is effected in the presence of sodium acetate under substantially anhydrous conditions.

16. A process for preparing a 14-hydroxy-3-O,N-(di-$R_1$-oxycarbonyl) normorphinone having the formula (M) set out in claim 1, which comprises reactively contacting a 3-O,N-(di-$R_1$-oxycarbonyl) normorphinone dienol acylate having the formula (L) set out in claim 1 with an aromatic or aliphatic monobasic or polybasic peroxy acid under reaction conditions effective for substituting an —OH group in the 14-position of the 3-O,N-(di-$R_1$-oxycarbonyl) normorphinone dienol acylate of formula (L) set out in claim 1, whereby a 14-hydroxy-3-O,N-(di-$R_1$-oxycarbonyl) normorphinone having the formula (M) set out in claim 1 is prepared.

17. A process according to claim 16, in which said peroxy acid is a substituted or unsubstituted perbenzoic acid wherein the substituent is chloro, bromo, iodo, fluoro or nitro; monoperphthalic acid; performic acid; peracetic acid; monopermaleic acid; trifluoroperacetic acid; or trichloroperacetic acid.

18. A process according to claim 16 or claim 17, in which said reaction conditions include reaction

(1) in the presence of

(A) an acid catalyst having a pKa of from 0 to 3,0; and

(B) a polar organic solvent which is a solvent for and substantially non-reactive with the 3-O,N-(di-$R_1$-oxycarbonyl) normorphinone dienol acylate having the formula (L) set out in claim 1, with said peroxy acid, and with said acid catalyst; said solvent being present in a solubilizing amount for each of the 3-O,N-(di-$R_1$-oxycarbonyl) normorphinone dienol acylate having the formula (L) set out in claim 1, said peroxy acid and said acid catalyst;

(2) in the substantial absence of water; and

(3) with at least about 1 gram-equivalent of said peroxy acid being present per gram-mole of the 3-O,N-(di-$R_1$-oxycarbonyl) normorphinone dienol acylate having the formula (L) set out in claim 1.

19. A process according to claim 18, in which said acid catalyst is oxalic acid, trichloracetic acid, phosphoric acid, trifluoroacetic acid, chloroacetic acid, maleic acid, methanesulfonic acid, or a mixture of two or more thereof.

20. A process according to claim 18 or claim 19, in which said peroxy acid is *m*-chloroperbenzoic acid, said acid catalyst is oxalic acid, and said solvent is glacial acetic acid.

**Patentansprüche**

1. Verfahren zur Herstellung von Noroxymorphon aus Morphin, bei dem man

(A) Morphin mit der Formel (I)

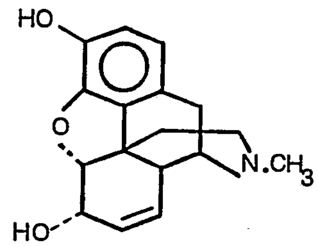

I

durch Umsetzung mit einem Halogenformiatester der Formel X—C(=O)$OR_1$, worin $R_1$ eine $C_1$-Bis $C_7$-Alkyl-, 1,1,1-Trichloräthyl-, Butenyl-, Benzyl-, Phenyl- oder Napththylgruppe und X ein Halogenatom ist, zu einem 3-O,N-(Di-$R_1$-oxycarbonyl)normorphin der Formel (J)

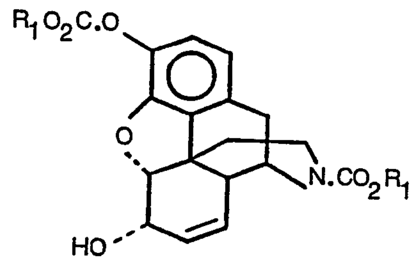

J

umsetzt, worin $R_1$ wie vorstehend angegeben definiert ist,

(B) das 3-O,N-(Di-$R_1$-oxycarbonyl)normorphin der Formel (J) durch Reaktion mit einem für die Oxidation allylischer OH-Gruppen zu Ketogruppen wirksamen Oxidationsmittel zu einem 3-O,N-(Di-$R_1$-oxycarbonyl)normorphin der Formel (K)

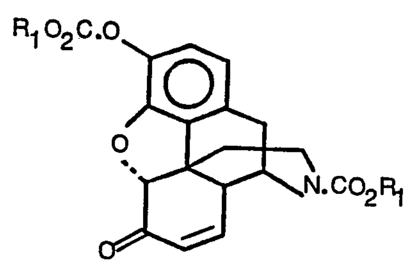

K

oxidiert, worin $R_1$ wie oben angegeben definiert ist,

(C) das 3-O,N-(Di-$R_1$-oxycarbonyl)normorphin der Formel (K) durch Umsetzung mit einem Acylierungsmittel, das unter Säureanhydriden der Formel $(R_2)_2O$ und Acylhalogeniden der Formel $R_2X'$ ausgewählt ist, worin $R_2$ eine Acylgruppe und X' ein Halogenatom bedeutet, zu einem 3-O,N-(Di-$R_1$-oxycarbonyl)normorphinon-dienolacylat der Formel (L) acyliert

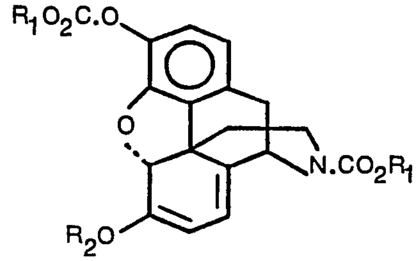

L

worin $R_1$ und $R_2$ die oben angegebene Bedeutung haben,

(D) das Dienolacylat der Formel (L) mit einem Peroxy-Oxidationsmittel zu einem 14-Hydroxy-3-O,N-(Di-$R_1$-oxycarbonyl)normorphinon mit der Formel (M) umsetzt,

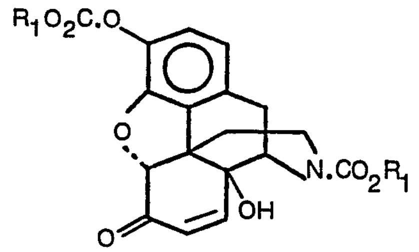

M

in der $R_1$ die oben angegebene Bedeutung hat,

(E) das 14-Hydroxy-3-O,N-(Di-$R_1$-oxycarbonyl)normorphinon der Formel (M) zu einem 3-O,N-(Di-$R_1$-oxycarbonyl)noroxymorphon der Formel (N)

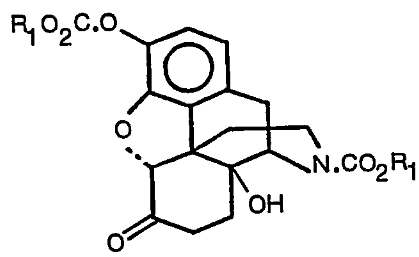

N

reduziert, in der $R_1$ wie oben angegeben definiert ist, und

(F) das 3-O,N-(Di-$R_1$-oxycarbonyl)noroxymorphon der Formel (N) zu Noroxymorphon der Formel (O)

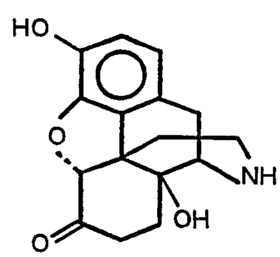

O

hydrolysiert.

2. Verfahren nach Anspruch 1, bei dem $R_1$ Methyl, Äthyl, Propyl, Heptyl, 1,1,1-Trichloräthyl, Vinyl, Butenyl, Phenyl, Benzyl oder Naphthyl ist.

3. Verfahren nach Anspruch 1 oder Anspruch 2, bei dem $R_1$ Äthyl ist.

4. Verfahren nach einem der Ansprüche 1 bis 3, bei dem $R_2$ Acetyl ist un ddas 3-O,N-(Di-$R_1$-oxycarbonyl)normorphinon-dienolacylat der Formel (L) ein 3-O,N-(Di-$R_1$-oxycarbonyl)normorphinon-dienolacetat ist.

5. 3-O,N-(Di-$R_1$-oxycarbonyl)normorphin mit der in Anspruch 1 angegebenen Formel (K).

6. Verbindung nach Anspruch 5, bei der $R_1$ in der Formel (K) Äthyl ist, so daß die genannte Verbindung 3-O,N-(Diäthoycarbonyl)normorphinon ist.

7. 3-O,N-(Di-$R_1$-oxycarbonyl)normorphinon-dienolacylat der in Anspruch 1 angegebenen Formel (L).

8. Verbindung nach Anspruch 7, in der $R_2$ in Formel (L) Acetyl ist, so daß die genannte Verbindung ein 3-O,N-(Di-$R_1$-oxycarbonyl)normorphinon-enolacetat ist.

9. Verbindung nach Anspruch 8, in der $R_1$ in Formel (L) Äthyl ist, so daß die genannte Verbindung 3-O,N-(Diäthoxycarbonyl)normorphinon-enolacetat ist.

10. 14-Hydroxy-3-O,N-(di-$R_1$-oxycarbonyl)normorphinon mit der in Anspruch 1 angegebenen Formel (M).

11. Verbindung nach Anspruch 10, in der $R_1$ in Formel (M) Äthyl ist, so daß die genannte Verbindung 14-Hydroxy-3-O,N-(diäthoxycarbonyl)normorphinon ist.

12. Verfahren zur Herstellung eines 3-O,N-(Di-$R_1$-oxycarbonyl)normorphinons der in Anspruch 1 angegebenen Formel (K), bei dem man ein 3-O,N-(Di-$R_1$-oxycarbonyl)normorphinon der in Anspruch 1 angegebenen Formel (J) mit einem für die Oxidation allylischer OH-Gruppen zu Ketogruppen wirksamen Oxidationsmittel reaktionsmäßig in Berührung bringt, wodurch eine Verbindung mit der in Anspruch 1 angegebenen Formel (K) hergestellt wird.

13. Verfahren nach Anspruch 12, bei dem das oxidationsmittel Chromtrioxid ist.

14. Verfahren zur Herstellung eines 3-O,N-(Di-$R_1$-oxycarbonyl)normorphinon-dienolacylats der in Anspruch 1 angegebenen Formel (L), bei dem man eine Verbindung der in Anspruch 1 angegebenen Formel (K) mit einem Acylierungsmittel, das ein Säureanhydrid der Formel $(R_2)_2O$ oder ein Acylhalogenid mit der Formel $R_2X'$ ist, worin $R_2$ eine Acylgruppe und X' ein Halogenatoms ist, reaktionsmäßig in Berührung bringt, wodurch ein 3-O,N-(Di-$R_1$-oxycarbonyl)normorphinon-dienolacylat der in Anspruch 1 angegebenen Formel (L) hergestellt wird.

15. Verfahren nach Anspruch 14, bei dem das Acylierungsmittel Essigsäureanhydrid ist und die Berührung in Gegenwart von Natriumcatat unter im wesentlichen wasserfreien Bedingungen erfolgt.

16. Verfahren zur Herstellung eines 14-Hydroxy-3-O,N-(Di-$R_1$-oxycarbonyl)normorphinons mit der in Anspruch 1 angegebenen Formel (M), bei dem man ein 3-O,N-(Di-$R_1$-oxycarbonyl)normorphinon-dienolacylat der in Anspruch 1 angegebenen Formel (L) mit einer aromatischen oder aliphatischen monobasischen oder polybasischen Peroxy-Säure unte Reaktionsbedingungen, die für die Substitution einer OH-Gruppe in der 14-Stellung des 3-O,N-(Di-$R_1$-oxycarbonyl)normorphinon-dienolacylats der in Anspruch 1 angebenen Formel (L) wirksam sind, reaktionsmäßig in Berührung bringt, wodurch ein 14-Hydroxy-3-O,N-(Di-$R_1$-oxycarbonyl)normorphinon der in Anspruch 1 angegebenen Formel (M) hergestellt wird.

17. Verfahren nach Anspruch 16, bei dem die Peroxysäure eine substituierte oder unsubstituierte Perbenzoesäure, in welcher der Substituent Chlor, Brom, Jod, Fuor oder Nitro ist, Monoperphthalsäure, Perameisensärue, Peressigsäure, Monopermaleinsäure, Trifluorperessigsäure oder Trichlorperessigsäure ist.

18. Verfahren nach Anspruch 16 oder 17, bei dem die Reaktionsbedingungen so sind, daß die Reaktion erfolgt

(1) in Gegenwart

(A) eines Säurekatalysators mit einem pKa-Wert von 0 bis 3,0 und

(B) eines polaren organischen Lösungsmittels, das ein Lösungsmittel für und im wesentlichen reaktionsträge gegenüber dem 3-O,N-(Di-$R_1$-oxycarbonyl)normorphinon-dienolacylat der in Anspruch 1 angegebenen Formel (L), der genannten Peroxy-Säure und dem genannten Säurekatalysator ist, wobei das Lösungsmittel in einer das 3-O,N-(Di-$R_1$-oxycarbonyl)normorphinon-dienolacylat der in Anspruch 1 angegebenen Formel (L), die genannte Peroxy-Säure und den genannten Säurekatalysator lösenden Menge vorliegt,

(2) im wesentlichen in Abwesenheit von Wasser und

(3) mit wenigstens etwa 1 Grammäquivalent der genannten Peroxy-Säure je Grammol des 3-O,N-(Di-$R_1$-oxycarbonyl)normorphinon-dienolacylat der in Anspruch 1 angegebenen Formel (L).

19. Verfahren nach Anspruch 18, bei dem der Säurekatalysator Oxalsäure, Trichloressigsäure Phosphorsäure, Trifluoressigsäure, Chloressigsäure, Maleinsäure, Methansulfonsäure oder ein Gemisch aus zwei oder mehreren diese Säuren ist.

20. Verfahren nach Anspruch 18 oder Anspruch 19, in dem die Peroxy-Säure m-Chlorperbenzoesäure, der Säurekatalysator Oxalsäure und das Lösungsmittel Eisessigsäure sind.

## Revendications

1. Procédé de préparation de la noroxymorphone à partir de la morphine, qui consiste:

(A) à transformer la morphine, de formule (I):

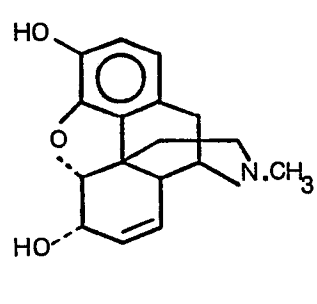

I

en la faisant réagir avec un haloformiate de formule X—C(=)$OR_1$, dans laquelle $R_1$ est un radical alkyle en $C_1$—$C_7$, 1,1,1-trichloréthyle, butényle, benzyle, phényle ou naphtyle et X est un atome d'halogène, en une 3-O,N-(di-$R_1$-oxycarbonyl) normorphine de formule (J):

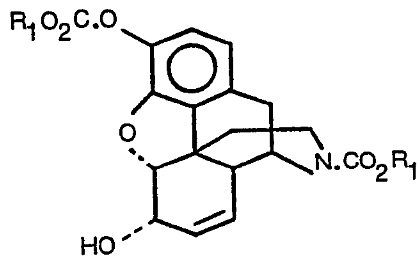

J

dans laquelle $R_1$ est tel que défini ci-dessus;

(B) à oxyder la 3-O,N-(di-$R_1$-oxycarbonyl) normorphine de formule (J), en la faisant réagir avec un agent oxydant ayant pour effet d'oxyder les groupements —OH allyliques en groupements cétoniques, pour former une 3-O,N-(di-$R_1$-oxycarbonyl)normorphinone de formule (K):

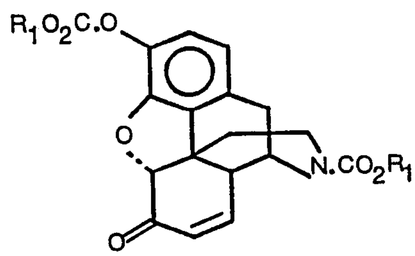

K

dans laquelle $R_1$ est tel que défini ci-dessus;

(C) à acyler la 3-O,N-(di-$R_1$-oxycarbonyl) normorphinone de formule (K), en la faisant réagir avec un agent d'acylation choisi parmi les anhydrides d'acide de formule $(R_2)_2$ O et les halogénures d'acyle de formule $R_2X'$, où $R_2$ est un radical acyle et X' un atome d'halogène, pour former un diénol acylate de 3-O,N-(di-$R_1$-oxycarbonyl) normorphinone, de formule (L):

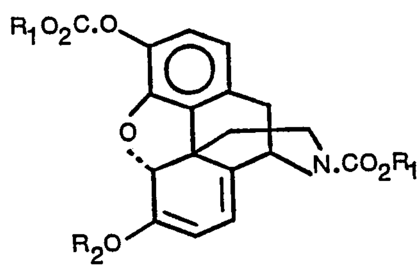

L

dans laquelle $R_1$ et $R_2$ sont tels que définis ci-dessus;

(D) à faire réagir le diénol acylate de formule (L) avec un agent peroxydant pour former une 14-hydroxy-3-O,N-(di-$R_1$-oxycarbonyl) normorphinone de formule (M):

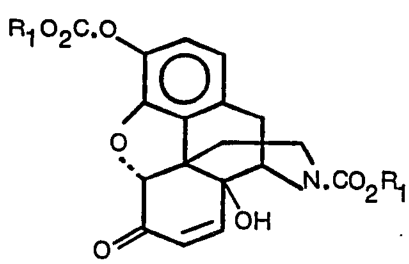

M

dans laquelle $R_1$ est tel que défini ci-dessus;

(E) à réduire la 14-hydroxy-3-O,N-(di-$R_1$-oxycarbonyl) normorphinone de formule (M) pour former une 3-O,N-(di-$R_1$-oxycarbonyl) noroxymorphone de formule (N):

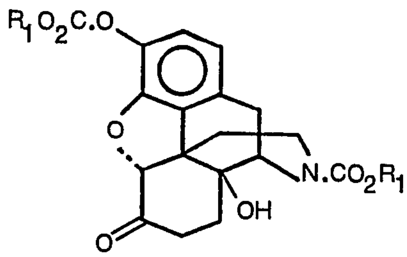

N

dans laquelle $R_1$ est tel que défini ci-dessus; et

(F) à hydrolyser la 3-O,N-(di-$R_1$-oxycarbonyl) noroxymorphone de formule (N) pour former la noroxymorphone de formule (O):

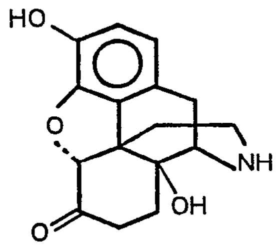

O

2. Procédé selon la revendication 1, dans laquelle $R_1$ est le radical méthyle, éthyle, propyle, heptyle, 1,1,1-trichloréthyle, vinyle, butényle, phényle, benzyle, ou naphtyle.

3. Procédé selon la revendication 1 ou la revendication 2, dans lequel $R_1$ est le radical éthyle.

4. Procédé selon l'une quelconque des revendications 1 à 3, dans lequel $R_2$ est le radicl acétyle et le diénol acylate de 3-O,N-(di-$R_1$-oxycarbonyl) normorphinone de formule (L) est un diénol acétate de 3-O,N-(di-$R_1$-oxycarbonyl) normorphinone.

5. 3-O,N-(di-$R_1$-oxycarbonyl) normorphinone ayant la formule (K) indiquée dans la revendication 1.

6. Composé selon la revendication 5, dans lequel $R_1$, dans la formule (K), et le radical éthyle, ledit composé étant la 3-O,N-(diéthoxycarbonyl) normorphinone.

7. Diénol acylate de 3-O,N-(di-$R_1$-oxycarbonyl) normorphinone ayant la formule (L) indiquée dans la revendication 1.

8. Composé selon la revendication 7, dans lequel $R_2$, dans la formule (L), est le radical acétyle, ledit composé étant un énol acétate de 3-O,N-(di-$R_1$-oxycarbonyl) normorphinone.

9. Composé selon la revendication 8, dans lequel $R_1$, dans la formule (L), est le radical éthyle, ledit composé étant l'énol acétate de 3-O,N-(diéthoxycarbonyl) normorphinone.

10. 14-Hydroxy-3-O,N-(di-$R_1$-oxycarbonyl) normorphinone ayant la formule (M) indiquée dans la revendication 1.

11. Composé selon la revendication 10, dans lequel $R_1$, dans la formule (M), est le radical éthyle, ledit composé étant la 14-hydroxy-3-O,N-(diethoxycarbonyl) normorphinone.

12. Procédé de préparation d'une 3-O,N-(di-$R_1$-oxycarbonyl) normorphinone ayant la formule (K) indiquée dans la revendication 1, qui consiste à mettre en contact réactif une 3-O,N-(di-$R_1$-oxycarbonyl) normorphine ayant la formule (J) indiquée dans la revendication 1, avec un agent oxydant ayant pour effet d'oxyder les groupements —OH allyliques en groupements cétoniques, un composé ayant la formule (K) indiquée dans la revendication 1 étant ainsi préparé.

13. Procédé selon la revendication 12, dans lequel l'agent oxydant es le trioxyde de chrome.

14. Procédé de préparation d'un diénol acylate de 3-O,N-(di-$R_1$-oxycarbonyl) normorphinone ayant la formule (L) indiquée dans la revendication 1, qui consiste à mettre en contact réactif un composé ayant la formule (K) indiquée dans la revendication 1, avec un agent d'acylation qui est un anhydride d'acide de formule $(R_2)_2O$ ou un halogénure d'acyle de formule $R_2X'$, où $R_2$ est un radical acyle et X' est un atome d'halogène, un diénol acylate de 3-O,N-(di-$R_1$-oxycarbonyl) normorphinone ayant la formule (L) indiquée dans la revendication 1 étant ainsi préparé.

15. Composé selon la revendication 14, dans lequel ledit agent d'acylation est l'anhydride acétique et le contact est effectué en présence d'acétate de sodium en milieu sensiblement anhydre.

16. Pocédé de préparation d'une 14-hydroxy-3-O,N-(di-$R_1$-oxycarbonyl) normorphinone ayant la formule (M) indiquée dans la revendication 1, qui consiste à mettre en contact réactif un diénol acylate de 3-O,N-(di-$R_1$-oxycarbonyl) normorphinone ayant la formule (L) indiquée dans la revendication 1, avec un peracide monobasique ou polybasique, aromatique ou aliphatique, dans des conditions de réaction permettant de substituer un groupement —OH en position 14 du diénol acylate de 3-O,N-(di-$R_1$-oxycarbonyl) normorphinone ayant la formule (L) indiquée dans la revendication 1, une 14-hydroxy-3-O,N-(di-$R_1$-oxycarbonyl) normorphinone ayant la formule (M) indiquée dans la revendication 1 étant ainsi préparée.

17. Procédé selon la revendication 16, dans lequel ledit peracide est un acide perbenzoïque éventuellement substitué par du chlore, du brome, de l'iode, du fluor ou un groupement nitro; l'acide monoperphtalique; l'acide performique; l'acide peracétique; l'acide monopermaléique; l'acide trifluoroperacétique; ou l'acide trichloroperacétique.

18. Procédé selon la revendication 16 ou la revendication 17, dans lequel lesdites conditions de réaction comprennent une réaction

(1) en présence de

(A) un catalyseur acide dont le pKa et de 0 à 3,0; et

(B) un solvant organique polaire qui est un solvant pour le diénol acylate de 3-O,N-(di-$R_1$-oxycarbonyl) normorphinone ayant la formule (L) indiquée dans la revendication 1, et est sensiblement inerte vis-à-vis de celui-ci, dudit peracide et dudit catalyseur acide; ledit solvant étant présent en quantité suffisante pour solubiliser le diénol acylate de 3-O,N-(di-$R_1$-oxycarbonyl) normorphinone ayant la formule (L) indiquée dans la revendication 1, ledit peacide et ledit catalyseur acide;

(2) pratiquement en l'absence d'eau; et

(3) en présence d'au moins 1 équivalent-gramme environ dudit peracide par molécule-gramme du diénol acyalte de 3-O,N-(di-$R_1$-oxycarbonyl) normorphinone ayant la formule (L) indiqué dan sla revendicaiton 1.

19. Procédé selon la revendication 18, dans lequel ledit catalyseur acide est l'acide oxalique, l'acide trichloracétique, l'acide phosphorique, l'acide trifluoracétique, l'acide chloracétique, l'acide maléique, l'acide méthanesulfonique, ou un mélange de deux ou plusieurs d'entre eux.

20. Procédé selon la revendication 18 ou la revendication 19, dans lequel ledit peracide est l'acide m-chloroperbenzoïque, ledit catalyseur acide est l'acide oxalique, et ledit solvant est l'acide acétique glacial.